# EUROPEAN PATENT SPECIFICATION

(11) **EP 2 708 599 B1**
(45) Date of publication and mention of the grant of the patent: **04.05.2016**
(21) Application number: 12869827.1
(22) Date of filing: 18.07.2012
(51) Int. Cl.: C12N 15/03, A61K 35/74, A61P 39/02, C12P 1/04, A23C 13/16, A23C 19/032, A23C 11/10, A23L 19/00, C12R 1/25

(54) **LACTOBACILLUS PLANTARUM ABLE TO RELIEVE LEAD TOXICITY AND USE THEREOF**
LACTOBACILLUS PLANTARUM ZUR LINDERUNG VON BLEIVERGIFTUNGEN UND SEINE VERWENDUNG
LACTOBACILLUS PLANTARUM SUSCEPTIBLE D'ENRAYER LA TOXICITÉ DU PLOMB ET SES APPLICATIONS

(30) Priority: 28.02.2012 CN 201210046323
(43) Date of publication of application: 19.03.2014
(73) Proprietor: Jiangnan University, Jiangsu 214122 (CN)
(72) Inventor: TIAN, Fengwei, Wuxi Jiangsu 214122 (CN); CHEN, Wei, Wuxi Jiangsu 214122 (CN); ZHAI, Qixiao, Wuxi Jiangsu 214122 (CN); ZHANG, Hao, Wuxi Jiangsu 214122 (CN); ZHAO, Jianxin, Wuxi Jiangsu 214122 (CN); WANG, Gang, Wuxi Jiangsu 214122 (CN); SONG, Yuanda, Wuxi Jiangsu 214122 (CN); ZHANG, Qiuxiang, Wuxi Jiangsu 214122 (CN); LIU, Xiaoming, Wuxi Jiangsu 214122 (CN); GUO, Min, Wuxi Jiangsu 214122 (CN); FAN, Daming, Wuxi Jiangsu 214122 (CN)
(74) Representative: ProI European Patent Attorneys
(86) International application number: PCT/CN2012/078799
(87) International publication number: WO 2013/127146

(56) References cited:
- WO-A1-2007/004966
- CN-A- 102 296 046
- ABOU-BAKER SALIM ET AL: "Effect of Lactic Acid Bacteria against Heavy Metals Toxicity in Rats", JOURNAL OF AMERICAN SCIENCE, vol. 7, no. 4, 1 January 2011 (2011-01-01) , pages 264-274, XP055153705, ISSN: 1545-1003
- HALTTUNEN ET AL: "Rapid removal of lead and cadmium from water by specific lactic acid bacteria", INTERNATIONAL JOURNAL OF FOOD MICROBIOLOGY, ELSEVIER BV, NL, vol. 114, no. 1, 3 February 2007 (2007-02-03), pages 30-35, XP005871967, ISSN: 0168-1605, DOI: 10.1016/J.IJFOODMICRO.2006.10.040
- IBRAHIM ET AL: "Probiotic bacteria as potential detoxification tools: assessing their heavy metal binding isotherms", CANADIAN JOURNAL OF MICROBIOLOGY, NRC RESEARCH PRESS, CA, vol. 52, no. 9, 1 January 2006 (2006-01-01), pages 877-885, XP008173414, ISSN: 0008-4166
- DATABASE WPI Week 200931 Thomson Scientific, London, GB; AN 2009-G01082 XP002741090, & CN 101 368 165 A (LIAONING MEDICAL COLLEGE) 18 February 2009 (2009-02-18)
- WANG, HAI-TAO ET AL.: 'Safe and Selective Compositions Screened for Specific Microbial Biosorption Capability to Heavy Metal Lead' FOOD SCIENCE vol. 28, no. 11, 30 December 2007, pages 390 - 396, XP008170760

## Description

### FIELD OF THE INVENTION

The invention particularly relates to a Lactobacillus plantarum capable of relieving lead toxicity and the application thereof.

### BACKGROUND ART

Lead is one kind of heavy metals extremely harmful to human health and is applied widely due to its unique physicochemical properties. As an important industrial material, lead has penetrated in all fields of human life; however, lead is one kind of metal elements poisonous to many organs without any physiological function in a human body, and the ideal blood lead level should be zero. With the rapid development of industry, agriculture, transportation, paint, press and electronic industry, air, soil, water and food are polluted by lead at different levels. Increasing lead contact and absorption because of long-time lead exposure causes lead accumulation in vivo. Lead can act on all systems and organs throughout the body, and its toxicity is related to forms and solubility of plumbic compound, but mainly injuries hemopoietic system, angiocarpy, digestive system, urinary system and immunologic system, thus having direct damage to human health, especially to nervous system, kidney, hemopoietic system and blood vessel. Lead-poisoned patients will develop symptoms of bellyache, diarrhea, vomiting, headache, dizziness, coma, etc., and take physical diseases such as vasospasm, liver and kidney damage, etc.

Various physiological diseases caused by lead accumulation and lead poisoning are usually treated by injecting chelating agents such as calcium disodium edetate, sodium dimercaptosuccinate and the like to expel lead in traditional therapies. For example, the Second version of Chinese Pharmacopoeia (2010) records that EDTANa2Ca acts as an antidote for heavy metal poisoning caused by lead, cadmium, etc. However, such medicant has certain toxicity and side effects. EDTANa2Ca is a wide-adaptability complexing agent with stronger renal toxicity and will expel via urine indispensable trace elements such as zinc, cuprum, manganese, ferrum and the like which are related to the activity of many enzymes, and therefore the high doses of EDTANa2Ca will endanger health. Sodium dimercaptosuccinate may cause disorder of the digestive system and symptoms of headache, nausea, aching limbs, etc. In view of many defects existing in traditional therapies, it is very necessary to develop a new intervention or therapy for lead accumulation and lead poisoning.

Lactobacillus is a general term for bacteria capable of fermenting carbohydrate to produce lactic acid and widely exits in naturally fermented dairy products, fermented plant food such as pickled vegetables, Chinese sauerkraut, silage and human intestines. Results of long-time scientific research show that probiotics represented by Llactobacillus are essential to a human body and have important physiological functions which mainly include preventing lactose intolerance, recovering intestinal colony balance and keeping health, anti-tumor and cancer prevention, controlling toxin levels in human bodies, protecting livers and enhancing detoxification functions of livers, etc. Therefore, the research on effects of Lactobacillus in relieving lead poisoning can further tap functions of Lactobacillus, develop Lactobacillus with higher health-protecting value and open up new ways and solutions for relieving lead poisoning by dietary methods.

Currently, some patent documents relate to lead-excretion health food and preparation methods thereof, for instance, the patent CN101933937A discloses a low molecular citrus pectin extract having natural chelating function and capable of expelling lead out of animal bodies; the patent CN101011432 relates to the use of extract of bracken flavones as medicament for lead expelling and lead poisoning alleviation; the patent CN1506070 provides a lead-expelling health product containing stachyose. However, there are less patent documents relating lactobacilli capable of relieving lead poisoning effects at present, for example, the patent CN101134945A discloses Klebsiella pneumoniae Z-KAE15 which has high lead tolerance and high lead adsorbability and can be applied to environmental bioengineering. In addition, the patent CN101252943A discloses some Lactobacillus plantarum strains used for increasing the absorption of Fe, Zn, Ca and Mg ions in a mammal model.

Therefore, it is very necessary to screen a Lactobacillus strain having function of relieving lead poisoning, prove that it has an excellent effect of relieving lead poisoning in animal models and develop the practical application thereof. The inventor eventually finishes the invention based on the prior art and a lot of experiments.

### SUMMARY OF THE INVENTION

### Object of the invention

The object of the invention is to provide a Lactobacillus plantarum CCFM8661.

The other object of the invention is to provide the application of the Lactobacillus plantarum CCFM8661.

### Technical solutions

The invention is realized by the following technical scheme.

The invention relates to a Lactobacillus strain which is identified as Lactobacillus plantarum CCFM8661 according to microbiological characteristics of morphological characteristics, culture characteristics and physiological and biochemical characteristics. The Lactobacillus plantarum strain has been deposited by China General Microbiological Culture Collection Center of Institute of Microbiology of Chinese Academy of Sciences located at NO. 1 Beichen West Road, Chaoyang District, Beijing, China, with the preservation number of CGMCC No.5494.

The Lactobacillus plantarum CCFM8661 has the following characteristics:
(1) Acid resistance; growing well in the environment condition of pH3.0-9.0 and surviving well under the condition of pH2.5;
(2) Excellent tolerance to lead ions after culture in lead-containing medium in vitro;
(3) High lead ions binding capacity after incubation in lead-containing aqueous solution in vitro;
(4) Effect of reducing lead contents in mice exposed to lead and relieving lead toxicity for mice exposed to lead.

The invention further relates to the application of the Lactobacillus plantarum CCFM8661 to preparation of pharmaceutical compositions and fermented foods capable of relieving lead toxicity.

According to one preferred embodiment of the invention, said pharmaceutical composition is composed of the Lactobacillus plantarum CCFM8661 cells and a pharmaceutically acceptable carrier.

According to another preferred embodiment of the invention, said Lactobacillus plantarum CCFM8661 preparation is powder prepared with bacterial culture containing the Lactobacillus plantarum CCFM8661 via the conventional freeze-drying preparation method or other methods and contains viable Lactobacillus plantarum CCFM8661 beyond 106CFU/mL.

According to another preferred embodiment of the invention, the pharmaceutically acceptable carrier is one or more carriers pharmaceutically used in common from the group consisting of filler, adhesive, wetting agent, disintegrant, lubricant and flavoring.

According to another preferred embodiment of the invention, said pharmaceutical composition is from the group consisting of granules, capsules, tablets, pills and oral liquid.

According to another preferred embodiment of the invention, said fermented foods include dairy products, soy bean products and fruit and vegetable products produced with starter containing the Lactobacillus plantarum CCFM8661.

According to another preferable embodiment of the invention, said starter is obtained as follows:
A. Preparation of culture medium: dissolving 10% of enzymatic hydrolysis skim milk, 0.5% of glucose, 1.5% of tryptone and 0.3% of yeast extract in water accounting for 87.7% of the total weight of the culture medium, adjusting the pH value to 6.8, and obtaining the culture medium;
B. Preparation of protectant: using water and material of protectant to prepare protectant containing 100g/L skim milk powder, 30mg/L glycerol, 100g/L maltodextrin, 150g/L trehalose and 10g/L L-sodium glutamate;
C. Inoculating the Lactobacillus plantarum CCFM8661 strains accounting for 2-4% of the weight of the culture medium in the culture medium sterilized for 8-12min at 110-120°C, culturing for 18h in the condition of 37°C, then washing with pH7.2 PBS 2-4 times , and resuspending in the protectant until reaching the concentration of 1010CFU/ml; and subsequently, pre-culturing the suspension for 60min at 37°C and then obtaining said starter via the freeze-drying method.

According to another preferred embodiment of the invention, said dairy products include cow milk, sour cream and cheese; said soy bean products include soy milk, lobster sauce and soybean sauce; and said fruit and vegetable products include products of cucumber, carrot, beet, celery and cabbage.

### DETAILED DESCRIPTION OF THE INVENTION

The invention relates to a Lactobacillus plantarum CCFM8661, and the Lactobacillus plantarum strain has been deposited by China General Microbiological Culture Collection Center of Institute of Microbiology of Chinese Academy of Sciences located at No.3, Courtyard 1, Beichen West Road, Chaoyang District, Beijing, China, with the preservation number of CGMCC No.5494.

According to the following screening standard, the inventor screens out the said Lactobacillus plantarum CCFM8661 from Chinese traditional foods such as pickle and fermented kumiss based on a lot of screening experiments and analysis and validation.

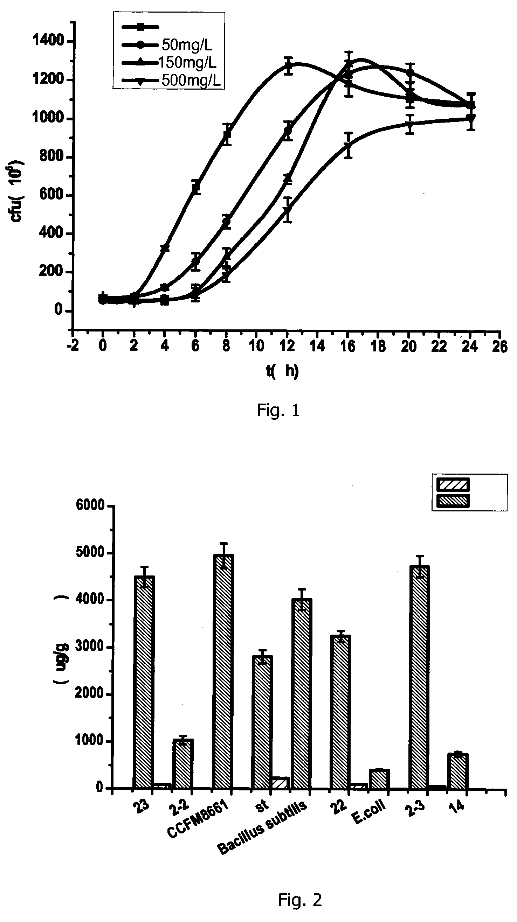

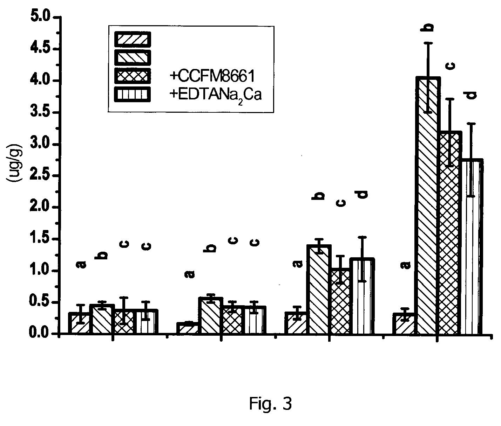

The characteristics of the Lactobacillus plantarum CCFM8661 will be described in details by combining related experiments and analysis and validation.
1. Acid resistance; the Lactobacillus plantarum grows well in the environment condition of pH3.0-9.0 and survives well under the condition of pH2.5.
   The cryopreserved Lactobacillus plantarum CCFM8661 of the invention is inoculated into a MRS culture medium (e.g. product of Qingdao Hope Bio-Technology Co.,Ltd) and cultured for 24h at 37°C, after 2-3 subcultures in MRS culture medium 1mL of Lactobacillus plantarum CCFM8661 culture is respectively inoculated into 19mL of MRS liquid medium with different pH values (3.0-9.0) and cultured for 24h at 37°C, determination of initial OD600 value and final OD600 value is carried out by which the strain concentration in the culture medium is measured so as to estimate the growth of the strain. The experimental results show that the Lactobacillus plantarum CCFM8661 grows well in the condition of pH3.0-9.0, and thus subsequent experiments can be carried out.
   Then the Lactobacillus plantarum CCFM8661 culture is obtained in the same way as above. The cells are washed with 1.0ml of pH7.2 PBS (phosphate buffer solution) twice and then resuspended in 1.0ml of pH7.2 PBS, the suspension is mixed with 9.0mL of pH2.5 artificial gastric juice and cultured at 37°C. Different samples are collected at the start (0h) and 3h for plate colony count via pour plate method using MRS agar culture medium. Afterwards the viable count is determined and the survival rate is calculated. The survival rate is the percentage of logarithmic values of viable counts at the third hour to the zero hour. In the invention, the strains with survival rate higher than 80% are screened for subsequent study.
   The experimental result shows that the Lactobacillus plantarum CCFM8661 has survival rate over 90% in the environment of artificial gastric juice. Thus it can be seen that the Lactobacillus plantarum CCFM8661 has acid resistance and can survive well in the condition of pH2-.5.
2. Excellent tolerance to lead ions after cultured in lead-containing medium in vitro The tolerance to lead ions in vitro of Lactobacillus strains can be studied through growth curves of the Lactobacillus strains in mediums with different lead contents. 0.20g of lead chloride is added into 1L of water in sterile conditions, obtaining lead aqueous solution with the concentration of 150mg/L. Such lead aqueous solution is used to dissolve MRS solid culture medium powder, obtaining MRS liquid culture medium with lead ion concentration of 150mg/L. Likewise, MRS liquid culture mediums with lead ion concentrations of 50mg/L and 500mg/L are prepared respectively. The Lactobacillus plantarum CCFM8661 solution in stationary phase is inoculated into the MRS liquid culture medium containing lead by 2% weight of the MRS liquid culture medium containing lead. During culture, standard plate count is carried out at 0h, 2h, 4h, 6h, 8h, 12h, 16h, 20h and 24h respectively to obtain the growth curves of the Lactobacillus plantarum CCFM8661 shown in FIG.1.
   The result of FIG.1 shows that the Lactobacillus plantarum CCFM8661 of the invention has an excellent tolerance to lead ions.
3. High lead ions binding capacity after incubation in lead-containing aqueous solution in vitro
   Seven Lactobacillus strains are screened from Chinese traditional foods such as pickle and fermented kumiss in sterile conditions according to the screening standard of acid resistance (viable in condition of pH3.0). The seven Lactobacillus strains are purified and activated along with control strains of one Escherichia coli strain and one Bacillus subtilis strain. The above strains are moved in 150mg/L lead solution until the finial concentration of the strains reaches to 1g/L. The sample containing the strains is cultured at 37°C for 1h, then centrifuged for 20min at 6000r/min by a centrifuge, and washed with sterile water and centrifuged once. Supernatant is separated and removed, pure nitric acid is added to the obtained cells, digestion is carried out for 20min in a microwave digestion oven, lead ion content in the obtained digestion solution is determined with an atomic absorption spectrometry according to the method proposed by Yeager et al (Yeager D.W. , Cholak J. , Henderson E.W. Determination of lead in biological and related materials by atomic absorption spectrometry. Environmental Science and Technology 1971 ; 5 : 1020-1022.) so as to define the lead binding capacity of each strain. The determination result is shown in FIG.2.
   It is clear from FIG.2 that compared with other tested strains and control strains the Lactobacillus plantarum CCFM8661 of the invention has the maximal lead adsorption amount, and therefore having strong lead ions binding capacity.
4. Effect of relieving lead toxicity for mice exposed to lead
   Forty 20-25g healthy male Kunming mice are randomly divided into four groups: a negative control group, a lead acetate model control group, a Lactobacillus plantarum CCFM8661 treatment group and an EDTANa2Ca positive treatment control group. The negative control group is fed with normal drinking water; the rest of the three groups are fed with 1g/L lead acetate solution to establish lead-exposure poisoned models, and then the Lactobacillus plantarum CCFM8661 treatment group is daily fed via gavage with 2.0×109CFU/mL CCFM8661 skim milk suspension prepared in the embodiment 3 of the specification, and the positive treatment control group is injected intraperitoneally with 5g/L EDTANa2Ca daily. Blood is collected when the experiment is finished and the mice are killed. Livers, stomachs and kidneys are taken out respectively and digested in the same way as described above, and then lead content is determined with the above mentioned atomic absorption spectrophotometry described by Yeager et al. The determination result is shown in FIG.3.

Through comparing lead contents in blood, livers, kidneys and stomachs of mice of lead-exposure poisoning model groups with that of mice of the group fed with lead and Lactobacillus plantarum CCFM8661 and comparing body antioxidant index (SOD and MDA), it is found that the Lactobacillus plantarum CCFM8661 of the invention can lower the lead contents in blood, livers, kidneys and stomachs of the mice and significantly recover the antioxidant index of the mice.

Lead in the environment usually enters in a human body via food and breath to cause acute or chronic toxic effects on digestive system, nervous system, respiratory system and immune system and frequently brings on diseases such as intestinal colic, anaemia and muscle paralysis, etc., and in severe cases encephalopathy occurs, sometimes leading to death. Therefore, diagnostic Criteria of Occupational Chronic Lead Poisoning GBZ37-2002 is usually used to determine whether a body is lead-poisoned or not at present.

In the invention the term "relieving lead toxicity" should be interpreted as a course of lightening or eliminating lead poisoning phenomena in a body. According to the Technical Standard for Testing & Assessment of Health Food issued by the Ministry of Health of China in 2003, experimental study proves that pharmaceutical composition containing the Lactobacillus plantarum CCFM8661 of the invention can reduce lead contents in blood, livers and kidneys of lead-poisoned mice, alleviate body's oxidative stress, relieve the pathological symptoms of lead-poisoned mice, increase the glutathione content in a mouse, and reduce the malondialdehyde content, thereby having the health function of promoting lead excretion.

The research results indicate that Lactobacillus plantarum CCFM8661 of the invention has the following characteristics:
(1) Acid resistance; growing well in the environment condition of pH3.0-9.0 and surviving well under the condition of pH2.5;
(2) Excellent tolerance to lead ions after culture in lead-containing medium in vitro;
(3) High lead ions binding capacity after incubation in lead-containing aqueous solution in vitro;
(4) Effect of reducing lead contents in mice exposed to lead and relieving lead toxicity for mice exposed to lead.

The Lactobacillus plantarum CCFM8661 of the invention has the following biological characteristics:
Strain characteristics: gram-positive, rod-shaped, 0.5-1.0µm in wide, 2-4µm in length, single, in pairs or chains, no spore formed, and round at both ends.

Colony characteristics: clear colonies formed on MRS culture medium, 0.3-2.0mm in diameter, round, regular edge, milk white, opaque, wet and smooth surface, and no generation of pigment.

Growth characteristics: the lowest growth temperature of the strain being 20°C, the highest growth temperature being 40°C, the prefer temperature for growth ranging between 30°C and 37°C, the lowest and the highest initial growth pH values being 9.0 and 2.5 respectively, and the prefer initial pH value for growth being 6.0.

The Lactobacillus plantarum CCFM8661 strain of the invention has relatively short lag phase, and comes into the exponential phase after about 4h and reaches to the stationary phase after 12h.

The preservation method of the Lactobacillus plantarum of the invention:
The original strain of the Lactobacillus plantarum is preserved in the form of 30% weight of glycerol suspension at -75°C, or in the form of frozen dry powder at 4°C for use.

The culture method and conditions of the Lactobacillus plantarum CCFM8661: it can be used after cultured in MRS culture medium for 18-36h under facultative anaerobic condition and at 37°C.

The invention further relates to the application of the Lactobacillus plantarum CCFM8661 to the preparations of pharmaceutical composition and fermented food capable of relieving lead toxicity.

The pharmaceutical composition is composed of the Lactobacillus plantarum CCFM8661 culture and a pharmaceutically acceptable carrier.

According to the invention, the Lactobacillus plantarum CCFM8661 culture is frozen dry powder prepared with the Lactobacillus plantarum liquid suspension via the conventional freeze-drying preparation method, or powder prepared via other methods such as a spray-drying method.

The Lactobacillus plantarum CCFM8661 culture contains viable Lactobacillus plantarum CCFM8661 beyond 106CFU/mL.

The content determination method of the Lactobacillus plantarum CCFM8661 adopts the MRS standard plate count method well known by those skilled in the art.

In the pharmaceutical composition, the amount of the Lactobacillus plantarum CCFM8661 accounts for 15-35% of the pharmaceutical composition by weight, preferably 18-32%, most preferably 20-30%.

According to the invention, the pharmaceutically acceptable carrier should be the conventional drug carrier, e.g. one or more carriers from the group consisting of filler, adhesive, wetting agent, disintegrant, lubricant and flavoring pharmaceutically used in common.

According to the invention, said filler should be interpreted in the sense of supplementary material diluent used for increasing the weight and volume of tablets to facilitate tabletting; or supplementary material absorbent used for absorbing surplus liquid in raw material.

Said filler is selected from the group consisting of starch, sucrose, lactose, calcium sulphate and microcrystalline cellulose.

Preferably, said filler is selected from the group consisting of starch, sucrose and microcrystalline cellulose.

More preferably, said filler is selected from the group consisting of starch and microcrystalline cellulose.

According to the invention, said wetting agent should be interpreted in the sense of liquid capable of wetting raw and supplementary materials of inviscid medicant and inducing viscosity of the medicant for granulation.

Said wetting agent is selected from the group consisting of water, ethanol, starch and syrup.

Preferably, said wetting agent is selected from the group consisting of water, ethanol and starch.

The amount of the wetting agent used in the invention is 0.1-3.0% of the total weight of the pharmaceutical composition.

According to the invention, said adhesive should be interpreted in the sense of a viscous substance added into raw medicant having no viscosity or insufficient viscosity for granulation.

Said adhesive is selected from the group consisting of cellulose derivatives, alginate, gelatin and polyvinyl pyrrolidone.

Preferably, said adhesive is selected from the group consisting of cellulose derivatives, gelatin and polyvinyl pyrrolidone.

More preferably, said adhesive is selected from the group of consisting of gelatin and polyvinyl pyrrolidone.

The amount of the adhesive used in the invention is 0.5-5.0% of the total weight of the pharmaceutical composition.

According to the invention, said disintegrant should be interpreted in the sense of a kind of supplementary material which can be added in tablets to promote the tablets to be rapidly disintegrated into fine particles in gastrointestinal fluid. It is well known that tablets due to being harder after compress are disintegrated very slowly to decrease therapeutic effects unless containing supplementary material having the function of promoting disintegration.

Said disintegrant is selected from the group consisting of sodium carboxymethyl starch, hydroxypropyl, crosslinked carboxymethyl cellulose, agar, calcium carbonate and sodium bicarbonate.

Preferably, said disintegrant is selected from the group consisting of sodium carboxymethyl starch, hydroxypropyl, crosslinked carboxymethyl cellulose, agar and sodium bicarbonate.

More preferably, said disintegrant is selected from the group consisting of sodium carboxymethyl starch, hydroxypropyl, crosslinked carboxymethyl cellulose and sodium bicarbonate.

The disintegrant used in the invention is 5.0-15.0% of the total weight of the pharmaceutical composition.

According to the invention, said lubricant should be interpreted in the sense of a kind of chemical substances capable of helping the increase of fluidity of tablets during granulation to prevent tablet material from adhering to the mold of a tablet machine, thereby being favorable of demoulding of tablets.

Said lubricant is selected from the group consisting of talc powder, calcium stearate, magnesium stearate, micropowder silica gel and polyethylene glycol.

Preferably, said lubricant is selected from the group consisting of talc powder, calcium stearate, magnesium stearate and polyethylene glycol.

More preferably, said lubricant is selected from the group consisting of talc powder and calcium stearate.

The lubricant used in the invention is 0.5-3.0% of the total weight of the pharmaceutical position.

According to the invention, said flavoring should be interpreted in the sense of medicinal auxiliary material used in medicine for improving or covering unpleasant smell or flavor to make strong bitterness or other peculiar smells such as pungency, irritation and the like imperceptible to patients.

Said flavoring is selected from the group consisting of sweetener of simple syrup, sucrose, lecithin, orange syrup or cherry syrup; aromatic of lemon, fennel or peppermint oil; mucilage of sodium alginate, arabic gum, gelatin, methyl cellulose or sodium carboxymethyl cellulose; and effervescent of the mixture of citric acid, tartaric acid and sodium bicarbonate.

Preferably, said flavoring is selected from the group consisting of sweetener of simple syrup, sucrose, orange syrup or cherry syrup; aromatic of lemon or peppermint oil; mucilage of sodium alginate, arabic gum, gelatin or sodium carboxymethyl cellulose; and effervescent of the mixture of tartaric acid and sodium bicarbonate.

More preferably, said flavoring is selected from the group consisting of sweetener of sucrose, orange syrup or cherry syrup; lemon oil aromatic; mucilage of sodium alginate or arabic gum; and effervescent of the mixture of tartaric acid and sodium bicarbonate.

The flavoring used in the invention is 0.5-2.0% of the total weight of the pharmaceutical composition.

The Lactobacillus plantarum CCFM8661 culture of the invention can be combined with pharmaceutically acceptable carrier or excipient to be prepared into various medicament forms such as granules, capsules, tablets, pills or oral liquid, wherein the pharmaceutically acceptable carrier or excipient can be selected depending on different medicament forms, and the carrier or excipient and the amount thereof are apparent and easy to determine for those skilled in the art of pharmacy.

The invention adopts methods and devices which are well-known and commonly used by those skilled in the art of pharmacy to prepare the granules, capsules, tablets, pills or oral liquid of the medicament of the invention.

Generally, said "medicament form" should be interpreted in the sense of applying to human being, and single medicament form contains specific active components with required content, such as the Lactobacillus plantarum CCFM8661 culture of the invention.

In the invention, said fermented foods include dairy products, soy bean products and fruit and vegetable products produced with starter containing the Lactobacillus plantarum CCFM8661.

One of preferable embodiments of the starter preparation method is as follows:
A. Preparation of culture medium: dissolving 10% of enzymatic hydrolysis skim milk, 0.5% of glucose, 1.5% of tryptone and 0.3% of yeast extract in water accounting for 87.7% of the total weight of the culture medium, adjusting the pH value to 6.8, and obtaining the culture medium;
B. Preparation of protectant: using water and material of protectant to prepare protectant containing 100g/L skim milk powder, 30mg/L glycerol, 100g/L maltodextrin, 150g/L trehalose and 10g/L L-sodium glutamate;
C. Inoculating the Lactobacillus plantarum CCFM8661 accounting for 2-4% of the weight of the culture medium in the culture medium sterilized for 8-12min at 110-120°C, culturing for 18h in the condition of 37°C, then washing with pH7.2 PBS 2-4 times , and resuspending in the protectant until reaching the concentration of 1010CFU/ml; and subsequently, pre-culturing the suspension for 60min at 37°C and then obtaining said starter via the freeze-drying method.

Said dairy products include milk, sour cream and cheese.

In the invention, said milk should be interpreted in the sense of cow milk, mare milk or reconstituted milk. Said reconstituted milk is prepared with milk powder and softened water accounting for 10-15% and 85-90% of the weight of the reconstituted milk respectively. Said milk powder is the product widely sold on market at present.

Said sour cream is prepared by fermented cream with Lactobacillus strain. Sour cream has more advantages than sweet cream of stronger aroma, higher yield of cream and lower risk of recontamination of microorganisms after sterilization because of harmful microorganisms being inhibited by the Lactobacillus strain.

Said cheese is a kind of foods having high nutritional value prepared by fermented milk.

Said soy bean products include soy milk, lobster sauce and soybean sauce which all are Chinese traditional foods or condiments.

Said fruit and vegetable products include products of cucumber, carrot, beet, celery and cabbage.

In the production of dairy products, soy bean products and fruit and vegetable products, the starter of the Lactobacillus plantarum CCFM8661 of the invention is used according to the following method:
Generally, in the conventional production of dairy products, soy bean products and fruit and vegetable products, the Lactobacillus plantarum CCFM8661 starter of the invention is inoculated in material to be treated at a conventional dosage and is fermented or survived at the temperature and pressure suitable for propagation of the Lactobacillus plantarum CCFM8661, the produced metabolites causes fermented products to process excellent properties of certain acidity, aroma or the like, thereby prolonging the storage time and improving the nutritional value and digestibility of the products.

### Beneficial effects

The Lactobacillus plantarum CCFM8661 of the invention has acid resistance, excellent tolerance to lead ions in vitro with capability of tolerating initial concentration of 150mg/L of lead ion solution and good binding capacity of lead ions, and can thus lower the lead contents in blood, livers, kidneys and stomachs of mice, significantly recover the antioxidant index of the mice and relieve the pathological symptoms of lead-poisoned mice. The Lactobacillus plantarum CCFM8661 is used for preparing pharmaceutical compositions and fermented foods capable of relieving lead poisoning, and has a very wide application prospect.

### BRIEF DESCRIPTION OF THE FIGURES

FIG.1 shows the growth curves of the Lactobacillus plantarum CCFM8661 in the culture medium containing lead ions with the initial concentration of 50mg/L, 150mg/L and 500mg/L;
FIG.2 shows the binding capacities of Lactobacillus strains, Bacillus subtilis and Escherichia coli to lead ions; wherein CCFM8661, 14 and ST-III represent Lactobacillus plantarum; 2-2 represents Lactobacillus buchneri; 2-3 represents Lactobacillus rhamnosus LGG; 22 represents Bifidobacterium; 23 represents Lactobacillus delbrueckii; Bacillus subtilis and Escherichia coli act as experimental control strains.
FIG.3 shows the effect of the Lactobacillus plantarum CCFM8661 on reducing the lead levels in blood, livers, kidneys and stomachs of mice exposed to lead; a, b, c, d, mean that groups represented by different letters have significant differences (P < 0.05).

### EXAMPLES

### Embodiment 1: experiment on the Lactobacillus plantarum CCFM8661 for tolerance to lead ions:

0.20g of lead chloride is added into 1L of water in sterile conditions, obtaining lead aqueous solution with the concentration of 150mg/L. Such lead aqueous solution is used to dissolve MRS solid culture medium mixture, obtaining MRS liquid culture medium with lead ion concentration of 150mg/L. MRS culture medium is the culture medium familiar to those skilled in the art and contains tryptone , yeast extract , glucose , sodium acetate, dibasic ammonium citrate, tween-80, magnesium sulfate and manganese sulfate, and pH value is 6.2-6.4.

MRS liquid culture mediums with lead ion concentrations of 50mg/L and 500mg/L are prepared respectively in the same way described above. The Lactobacillus plantarum CCFM8661 culture in stationary phase is inoculated into the MRS liquid culture medium containing lead by 2% weight of the MRS liquid culture medium containing lead, cultured at 37°C, sampled at 0h, 2h, 4h, 6h, 8h, 12h, 16h, 20h and 24h respectively for colony counting. The obtained growth curves of the Lactobacillus plantarum CCFM8661 are shown in FIG.1. In addition, 1mL of the Lactobacillus plantarum solution is inoculated into lead-free MRS culture medium and cultured in the same condition, and the result is of a blank control group and also listed in FIG.1.

FIG.1 shows that in the environment of lead ion concentration of 150mg/L, the growth of the Lactobacillus plantarum CCFM8661 is inhibited in the beginning but rapidly increases subsequently to even exceed the blank control group at 24h, indicating that the Lactobacillus plantarum CCFM8661 has an excellent tolerance to lead ions.

### Embodiment 2: experiment on the lead binding capacity of Lactobacillus plantarum CCFM8661:

According the screening standard of acid resistance (viable under the condition of pH3.0), seven Lactobacillus strains are screened from Chinese traditional foods such as pickle and fermented kumiss. The seven Lactobacillus strains are purified and activated in sterile conditions along with control strains of one Escherichia coli strain and one Bacillus subtilis strain, wherein the Lactobacillus strains are purified and activated in MRS culture medium, and the Escherichia coli and Bacillus subtilis in LB culture medium which is well known by those skilled in the art and contains tryptone , yeast extract , NaCl and agar, with pH7.0. In addition, the Escherichia coli and Bacillus subtilis further need to be placed in a shaking incubator for aerobic culture for 12h and 16h at 37°C respectively. The activated bacteria solution is oscillated and centrifuged for 15min at 6000r/min. The obtained cells are moved into a container containing the solution with lead ion concentration of 150mg/L, while a blank control group is moved into deionized water. The volume of liquid is determined according to the mass of the cells to obtain final concentration of the cells of 1g/L. Then the sample is cultured for 1h at 37°C, centrifuged for 20min at 6000r/min by a Beckman centrifuge, washed with sterile water and centrifuged again. Supernatant is separated and removed, pure nitric acid is added to the obtained cells, and the digestion is carried out for 20min in a microwave digestion oven. Lead ion content in the obtained digestion solution is determined with an atomic absorption spectrometry according to the method proposed by Yeager et al (Yeager D.W. , Cholak J. , Henderson E.W. Determination of lead in biological and related materials by atomic absorption spectrometry. Environmental Science and Technology 1971 ; 5 : 1020-1022.) so as to define the adsorption capacity of each strain.

The experiment result is shown in FIG.2. FIG.2 shows that there is a large difference in lead adsorption capacity among different strains. The Lactobacillus plantarum CCFM8661 has the strongest binding capacity of 4955.1µg/g lead ions, and the Escherichia coli has the poorest adsorption capacity for lead ions of only 398µg/g lead ions, which is far lower than the Lactobacillus plantarum CCFM8661.

### Embodiment 3: experiment on tolerance dose of the Lactobacillus plantarum CCFM8661 administered to mice via gavage:

The frozen dried powder of the Lactobacillus plantarum CCFM8661 is suspended in skim milk powder to form suspension with the concentration of 2.0×109CFU/mL. The strain suspension is administered to ten healthy male Kunming mice with weights at about 20g daily via gavage. Observation is carried out daily for one week, and the death and weight are recorded. The experimental result is listed in Table 1.

**Table 1: The change of mice weight fed with the Lactobacillus plantarum CCFM8661 with the concentration of 2.0×109 CFU/mL**

| Time(days) | 1 | 2 | 3 | 4 | 5 | 6 | 7 |
|---|---|---|---|---|---|---|---|
| Weight(g) | 21.2±1.5 | 21.9±2.1 | 22.5±2.2 | 23.5±2.1 | 24.2±1.9 | 24.9±1.9 | 25.7±1.8 |
| Death rate | - | - | - | - | - | - | - |

| | | | | | | | |
|---|---|---|---|---|---|---|---|
| Note: "-": no death | | | | | | | |

The result in the Table 1 shows that the Lactobacillus plantarum CCFM8661 with the concentration of 2.0×109 CFU/mL has no significant effect on mice, while the weight of mice increases and no death is observed. The mice have no remarkable pathological symptoms in appearance.

### Embodiment 4: effect of the Lactobacillus plantarum CCFM8661 on reducing the lead level in mice exposed to lead:

Forty 20-25g healthy male Kunming mice are randomly divided into four groups: a negative control group, a lead acetate model control group, a Lactobacillus plantarum CCFM8661 treatment group and a calcium disodium edetate positive treatment control group. The negative control group is fed with normal drinking water; the rest of the three groups are fed with 1g/L lead acetate solution to establish lead-exposure poisoned models, and then the Lactobacillus plantarum CCFM8661 treatment group is daily fed to mice via gavage with 2.0×109 CFU/mL CCFM8661 suspension prepared by the embodiment 3 of the specification, and the positive treatment control group is injected intraperitoneally with 5g/L EDTANa2Ca medicament daily. Blood is collected when the experiment is finished and the mice are killed, livers, stomachs and kidneys are taken out respectively and digested in the same way as described above, and then lead content is determined with the atomic absorption spectrophotometry used in embodiment 2.

The experimental result is shown in FIG.3. From FIG.3 we can see that lead contents in blood, livers, stomachs and kidneys of the mice of the Lactobacillus plantarum CCFM8661 treatment group and the EDTANa2Ca positive treatment control group are higher than the negative control group but lower than that of the lead model control group. Compared with the lead model control group, the lead contents in blood and organs of mice of the Lactobacillus plantarum CCFM8661 treatment group decrease significantly (P < 0.05), which indicates the interference of the Lactobacillus plantarum CCFM8661 has the function of reducing lead contents in mice exposed to lead.

### Embodiment 5: Recovery effect of the Lactobacillus plantarum CCFM8661 on oxidative damage caused by lead exposure:

Forty 20-25g healthy male Kunming mice are randomly divided into four groups: a negative control group, a lead acetate model control group, a Lactobacillus plantarum CCFM8661 treatment group and an EDTANa2Ca positive treatment control group. The negative control group is fed with normal drinking water; the rest of the three groups are fed with 1g/L lead acetate solution to establish lead poisoned models, and then the Lactobacillus plantarum CCFM8661 treatment group is daily administered to mice via gavage with 2.0×109 CFU/mL CCFM8661 suspension prepared in the embodiment 3 of the specification, and the positive treatment control group is injected intraperitoneally with 5g/L EDTANa2Ca medicament daily. Blood is collected when the experiment is finished and the mice are killed. Livers are taken out, and levels of glutathione (GSH) and malondialdehyde (MDA) are determined by the method of Ellmam (Ellmam, G.C. Tissue sulfhydryl groups. Archives of biochemistry and biophysics 82(1) : 70-77.) and with a malondialdehyde kit produced by Nanjing Jiancheng Bioengineering Institute.

The experimental result is shown in Table 2.

**Table 2: Recovery effect of the Lactobacillus plantarum CCFM8661 of the invention on oxidative damage caused by lead poisoning**

| Group (liver) | GSH(mg/g protein) | MDA(nmol/mg protein) |
|---|---|---|
| Control group | 2.69±0.4. | 0.11±0.04 |
| Lead only | 1.63±0.75a | 0.18±0.03a |
| Lead+CCFM8661 | 2.46±0.71b | 0.16±0.04b |
| Lead+ EDTANa2Ca | 2.15±0.50c | 0.15±0.03c |

| | | |
|---|---|---|
| Note: a: compared with the blank control group P < 0.05; compared with the lead model group P < 0.05; compared with the lead model P < 0.05 | | |

The result in Table 2 shows that in the sample of mice liver of the Lactobacillus plantarum CCFM8661 treatment group, GSH content is higher than that of the lead model group but lower than the blank control group; MDA content is lower than the lead model control group but higher than the blank control group. Compared with the lead model control group, both GSH content and MDA content of the Lactobacillus plantarum CCFM8661 treatment group have significant differences (P < 0.05). Glutathione (GSH) is an important antioxidant in a living body, while malondialdehyde (MDA) is an end product of peroxidation between free radicals and lipid and acts as a marker of oxidative stress injury in the host. As shown in Table 2, compared with the blank control group, the glutathione (GSH) concentration of the lead model group is significantly reduced, and the malondialdehyde (MDA) content increases obviously, which fully proves that lead exposure causes oxidative stress reaction in the mouse body. However, compared with the lead model group, in the Lactobacillus plantarum CCFM8661 treatment group the glutathione content increases significantly, and the malondialdehyde content decreases obviously, which clearly indicates that the treatment with the Lactobacillus plantarum CCFM8661 of the invention really relieves oxidative damage caused by lead poisoning.

The above animal experiments show that the Lactobacillus plantarum CCFM8661 of the invention can significantly reduce lead content in blood and organs of mice exposed to lead and recover the antioxidant index of the mice, thus effectively relieving lead toxicity effects.

### Application embodiment 1: preparation of cow milk containing the Lactobacillus plantarum CCFM8661:

Raw skim milk is sterilized at 95°C for 20 min and then cooled to 4°C, in which starter of the Lactobacillus plantarum CCFM8661 described in the specification is added subsequently until the concentration is beyond 106CFU/ml, and is refrigerated at 4°C, obtaining the cow milk containing the Lactobacillus plantarum CCFM8661.

### Application embodiment 2: preparation of soy milk containing the Lactobacillus plantarum CCFM8661:

Soybeans are soaked in soft water for 2h at 80°C, and then soybean skin is removed. Subsequently, draining off soaking water is followed, then adding boiling water for pulping, and the temperature is kept beyond 80°C for 12min. The obtained slurry is filtered with a 150-mesh sieve and centrifuged for separation, the centrifugal liquid obtained is crude soy milk which then is heated to 140-150°C, the hot soy milk is rapidly led into a vacuum cooling chamber for vacuum pumping, and odorous substance in the crude soy milk is rapidly discharged along with steam. After vacuum degassing, the crude soy milk is cooled to about 37°C, in which the Lactobacillus plantarum CCFM8661 starter of the invention is inoculated subsequently until the concentration thereof is beyond 106CFU/ml, and is refrigerated at 4°C, obtaining the soy milk containing the Lactobacillus plantarum CCFM8661.

### Application embodiment 3: preparation of fruit and vegetable drinks containing the Lactobacillus plantarum CCFM8661:

Fresh vegetable is selected to be washed and juiced, then undergoes high-temperature instant sterilization at the high temperature of 140°C for 2 seconds and immediately cooled to about 37°C, in which the Lactobacillus plantarum CCFM8661 starter prepared according to the invention is inoculated subsequently until the concentration thereof is beyond 106CFU/ml, and is refrigerated at 4°C, obtaining the fruit and vegetable drinks containing the Lactobacillus plantarum CCFM8661.

### Application embodiment 4: preparation of capsule products containing the Lactobacillus plantarum CCFM8661:

The Lactobacillus plantarum CCFM8661 of the invention is cultured in MRS culture medium for 24h, then centrifuged for 20min in the condition of 4°C and 40000r/min and washed with pH7.2 PBS twice, and the cells thereof is resuspended in sterilized skim milk until the final concentration of the cells reaches to 1×1010-3×1010CFU/mL. The bacterial suspension is added in sodium alginate solution with weight percentage of 3% for fully agitation until the cells are evenly dispersed in the sodium alginate solution, and the mixture liquid is squeezed into calcium chloride solution with weight percentage of 2% to form colloidal granules which is then filtered and collected after static solidification for 30min. The collected colloidal granules undergoes freeze-drying for 48h to form powder containing the Lactobacillus plantarum CCFM8661 which is then enclosed in pharmaceutical capsules now available on the market, obtaining the said capsule products.

### Application embodiment 5: using the Lactobacillus plantarum CCFM8661 to prepare starter used for producing dairy products, soy bean products and fruit and vegetable products:

The Lactobacillus plantarum CCFM8661 accounting for 3% of the weight of the culture medium is inoculated in the culture medium sterilized for 10min at 115°C, wherein the culture medium consists of 10% of enzymatic hydrolysis skim milk, 0.5% of glucose, 1.5% of tryptone , 0.3% of yeast extract and the rest of water in the percentage of the total weight of the culture medium, with pH value of 6.8. Subsequently, the culture medium inoculated with the Lactobacillus plantarum CCFM8661 is cultured for 18h in the condition of 37°C, then washed with pH7.2 PBS twice, and resuspended in the protectant to 1010CFU/ml. Said protectant contains 100g/L skim milk powder, 30mg/L glycerol, 100g/L maltodextrin, 150g/L trehalose and 10g/L L-sodium glutamate.

Subsequently, the suspension is pre-cultured for 60min at 37°C and then prepared into the starter of the dairy products, soy bean products and fruit and vegetable products via the freeze-drying method.

### Application embodiment 6: preparing fermented milk with the Lactobacillus plantarum CCFM8661 of the invention:

After added sugar is melted in fresh milk, the milk is homogenized in the condition of 65°C and 20MPa and sterilized for 5min at 95°C and then cooled to 35°C, and mixed bacteria is added into the milk. Said mixed bacteria consists of the Lactobacillus plantarum CCFM8661 of the invention, commercial powder starter Lactobacillus bulgaricus and commercial powder starter Streptococcus thermophilus in the mass ratio of 1:1:1, with the inoculum size being 0.03% of the total weight of the fresh milk. After well mixing, the milk is fermented by keeping the temperature of 35°C, and refrigerated for 24h at 4°C after curding, obtaining the fermented milk.

### Application embodiment 7: preparing tablets with the Lactobacillus plantarum CCFM8661 of the invention:

25.7 weight portion of bacteria powder of the Lactobacillus plantarum CCFM8661 of the invention prepared by the freeze-drying method, 55.0 weight portion of starch, 4.5 weight portion of cellulose derivatives , 12.0 weight portion of sodium carboxymethyl starch, 0.8 weight portion of talc powder, 1.0 weight portion of sucrose and 1.0 weight portion of water are respectively weighed and mixed to be prepared into wet granules in the conventional way, then tabulated with a tablet press machine for example produced by Zhongnan Pharmaceutical Machinery Factory, and dried in a small-sized drug dryer produced for example by Qingzhou City Yikong Chinese Traditional Medicine Co., Ltd , obtaining the tablets of the invention after repacking.

### Application embodiment 8: preparing pills with the Lactobacillus plantarum CCFM8661 of the invention:

32.2 weight portion of bacteria powder of the Lactobacillus plantarum CCFM8661 of the invention prepared by the freeze-drying method, 48.0 weight portion of microcrystalline cellulose, 4.5 weight portion of polyvinyl pyrrolidone, 10.0 weight portion of calcium carbonate, 2.8 weight portion of magnesium stearate, 1.3 weight portion of lecithin and 1.2 weight portion of ethanol are respectively weighed and mixed evenly, and conventional dose of refined honey is added in the mixture, obtaining the pills of the invention.

## Claims

1. A Lactobacillus plantarum CCFM8661 which has been deposited on November 29, 2011 by China General Microbiological Culture Collection Center of Institute of Microbiology, with the preservation number of CGMCC No.5494.

2. The Lactobacillus plantarum CCFM8661 according to claim 1, **characterized by** the following characteristics:
(1) Acid resistance; growing well in the environment condition of pH3.0-9.0 and surviving well under the condition of pH2.5;
(2) Excellent tolerance to lead ions after culture in lead-containing medium in vitro;
(3) High lead ions binding capacity after incubation in lead-containing aqueous solution in vitro;
(4) Effect of reducing lead contents in mice exposed to lead and relieving lead toxicity for mice exposed to lead.

3. The application of the Lactobacillus plantarum CCFM8661 according to claim 1 to the preparation of pharmaceutical compositions and fermented foods capable of relieving lead toxicity.

4. The application according to claim 3, **characterized in that** said pharmaceutical composition is composed of the Lactobacillus plantarum CCFM8661 and a pharmaceutically acceptable carrier.

5. The application according to claim 4, **characterized in that** said Lactobacillus plantarum CCFM8661 culture is powder prepared with bacterial suspension containing the Lactobacillus plantarum CCFM8661 via the conventional freeze-drying preparation method or other methods and contains viable Lactobacillus plantarum CCFM8661 beyond 106CFU/mL.

6. The application according to claim 4, **characterized in that** the pharmaceutically acceptable carrier is one or more carriers pharmaceutically used in common from the group consisting of filler, adhesive, wetting agent, disintegrant, lubricant and flavoring.

7. The application according to claim 3 or 4, **characterized in that** said pharmaceutical composition is from the group consisting of granules, capsules, tablets, pills and oral liquid.

8. The application according to claim 3, **characterized in that** said fermented foods include dairy products, soy bean products and fruit and vegetable products produced with starter containing the Lactobacillus plantarum CCFM8661.

9. The application according to claim 8, **characterized in that** the preparation of said starter comprises the following steps of:
A. Preparation of culture medium: 10% of enzymatic hydrolysis skim milk, 0.5% of glucose, 1.5% of tryptone and 0.3% of yeast extract are dissolved in water accounting for 87.7% of the total weight of the culture medium, the pH value is adjusted to 6.8, and saidculture medium is obtained;
B. Preparation of protectant: using water and material of protectant to prepare protectant containing 100g/L skim milk powder, 30mg/L glycerol, 100g/L maltodextrin, 150g/L trehalose and 10g/L L-sodium glutamate;
C. Inoculating the Lactobacillus plantarum CCFM8661 accounting for 2-4% of the weight of the culture medium in the culture medium sterilized for 8-12min at 110-120°C, culturing for 18h in the condition of 37°C, then washing with pH7.2 PBS 2-4 times , and resuspending in the protectant until reaching the concentration of 1010CFU/ml; and subsequently, pre-culturing the suspension for 60min at 37°C and then obtaining said starter via the freeze-drying method.

10. The application according to claim 8, **characterized in that** said dairy products include cow milk, sour cream and cheese; said soy bean products include soy milk, lobster sauce and soybean sauce; and said fruit and vegetable products include products of cucumber, carrot, beet, celery and cabbage.

## Patentansprüche

1. Lactobacillus plantarum CCFM8661, welches am 29. November 2011 vom allgemeinen chinesischen mikrobiologischen Kultursammelzentrum des Instituts für Mikrobiologie mit der Konservierungsnummer von CGMCC Nr. 5494 hinterlegt wurde.

2. Lactobacillus plantarum CCFM8661 nach Anspruch 1, **gekennzeichnet durch** folgende Merkmale:
(1) Säurebeständigkeit; gutes Wachstum bei Umgebungsbedingungen mit einem pH-Wert von 3,0 bis 9,0 und gute Überlebenschancen bei Umgebungsbedingungen mit einem pH-Wert von unter 2,5 überleben;
(2) ausgezeichnete Toleranz gegenüber Schwerionen nach Zuführen der Kultur in bleihaltiges Medium im Reagenzglas;
(3) Hohe Schwerionenbindungskapazität nach Inkubation in bleihaltiger wässriger Lösung im Reagenzglas;
(4) Reduzierte Wirkung der Bleigehalts auf Mäuse, welche dem Blei ausgesetzt sind, und Linderung der toxischen Wirkung von Blei, welchem Mäuse ausgesetzt sind.

3. Anwendung des Lactobacillus plantarum CCFM8661 nach Anspruch 1 zur Herstellung von pharmazeutischen Zusammensetzungen und fermentierten Lebensmitteln geeignet zur Linderung der Bleitoxizität.

4. Anwendung nach Anspruch 3, **dadurch gekennzeichnet, dass** die pharmazeutische Zusammensetzung aus dem Lactobacillus plantarum CCFM8661 und einem pharmazeutisch verträglichen Träger gebildet ist.

5. Anwendung nach Anspruch 4, **dadurch gekennzeichnet, dass** die Lactobacillus plantarum CCFM8661-Kultur ein Pulver ist, welches mittels eines herkömmlichen Gefriertrocknungsherstellungsverfahrens oder anderer Verfahren mit Bakteriensuspension bereitet wird, welche das Lactobacillus plantarum CCFM8661 enthält, und welches tragfähiges Lactobacillus plantarum CCFM8661 über 106CFU/mL enthält.

6. Anwendung nach Anspruch 4, **dadurch gekennzeichnet, dass** der pharmazeutisch verträgliche Träger ein oder mehrere Trägerstoffe sind, welche pharmazeutisch gemeinsam von der Gruppe bestehend aus Füllstoff, Klebstoff, Netzmittel, Sprengmittel, Gleitmittel und Aroma verwendet werden.

7. Anwendung nach Anspruch 3 oder 4, **dadurch gekennzeichnet, dass** die pharmazeutische Zusammensetzung aus der Gruppe der Granulate, Kapseln, Tabletten, Pillen und Flüssigkeiten zum oralen Einnehmen besteht.

8. Anwendung nach Anspruch 3, **dadurch gekennzeichnet, dass** fermentierte Lebensmittel Milchprodukte, Sojaprodukte und Obst und Gemüseprodukte umfasst, welche mit einem Starter, der den Lactobacillus plantarum CCFM8661 enthält, produziert werden.

9. Anwendung nach Anspruch 8, **dadurch gekennzeichnet, dass** die Herstellung der Starter folgende Verfahrensschritte umfasst:
A. Herstellung des Kulturmediums: 10% enzymatisch hydrolisierter Magermilch, 0,5% Glucose, 1,5% Trypton und 0,3% Hefeextrakt werden in Wasser mit 87,7% des Gesamtgewichts des Kulturmediums gelöst, wobei der pH-Wert auf 6,8 eingestellt und das Kulturmedium erhalten werden;
B. Herstellung von Schutzhilfsmittel: Wasser und Material zum Schützen zur Herstelleung des Schutzhilfsmittels, welches enthält: 100g/l Magermilchpulver, 30 mg/l Glycerin, 100 g/l Maltodextrin, 150 g/L Trehalose und 10 g/l L-Natriumglutamat;
C. Okulieren des Lactobacillus plantarum CCFM8661 mit 2 bis 4% des Gewichts des Kulturmediums in das Kulturmedium, welches für 8 bis 12min bei 110 bis 120 °C sterilisiert wird, Kultivierung für 18 Stunden unter der Bedingung von 37 °C, dann 2 bis 4-maliges Waschen bei pH-Wert von 7,2 PBS und Resuspendieren in dem Schutzhilfsmittel bis die Konzentration an 1010CFU/ml erreicht; und anschließendes Vorkultivieren der Suspension für 60 min bei 37 °C und dann Erhaltens des Starters über das Gefriertrocknungsverfahren.

10. Anwendung gemäß Anspruch 8, daß die Milchprodukte Kuhmilch, Sauerahm und Käse umfassen; dass die Sojaprodukte Sojamilch, Hummersauce und Sojasauce umfassen; und dass Obst und Gemüseprodukte Produkte aus Gurken, Karotten, Rüben, Sellerie und Kohl umfassen.

## Revendications

1. Lactobacillus plantarum CCFM8661, la souche a été déposée au China General Microbiological Culture Collection Center (CGMCC) de l'Institute de Microbiologie de l'Académie chinoise des sciences, en 29. novembre 2011, avec le numéro de préservation CGMCC No. 5494.

2. Lactobacillus plantarum CCFM8661, selon la revendication 1, **caractérisé par**
(1) une résistance à l'acide, les bactéries poussent bien dans des conditions d'environnement de pH 3,0-9,0, et survivre bien au pH de 2,5;
(2) une tolérance excellente aux ions de plomb, après avoir été cultivées in vitro dans un milieu contenant du plomb.
(3) une capacité de liaison élevée pour des ions de plomb, après avoir été mis en incubation in vitro dans une solution aqueuse contenant du plomb
(4) l'effet de réduire la teneur en plomb dans des souris, qui ont été exposés au plomb, et l'effet de soulager la toxicité du plomb chez des souris, qui ont été exposés au plomb.

3. Utilisation de Lactobacillus plantarum CCFM8661, selon la revendication 1, pour la préparation des compositions pharmaceutiques et des aliments fermentés, que sont capable de soulager la toxicité du plomb.

4. Utilisation de Lactobacillus plantarum CCFM8661, selon la revendication 3, **caractérisé en ce que** ces compositions pharmaceutiques sont composées des bactéries de Lactobacillus plantarum CCFM8661 et un excipient pharmaceutiquement acceptable.

5. Utilisation de Lactobacillus plantarum CCFM8661, selon la revendication 4, **caractérisé en ce que** ces bactéries Lactobacillus plantarum CCFM8661 sont préparées sous forme de poudre, et sont préparées à partir d'une suspension bactérienne, contenant des Lactobacillus plantarum CCFM8661, par le procédé de préparation classique de lyophilisation ou d'autres procédés, et que Lactobacillus plantarum CCFM8661 est contenue viable et au-delà de 106 UFC/ml.

6. Utilisation de Lactobacillus plantarum CCFM8661, selon la revendication 4, **caractérisé en ce que** l'excipient pharmaceutiquement acceptable est un ou plusieurs d'excipient pharmaceutiquement, utilisé couramment, et de le groupe constitué par un remplissage, un adhésif, un agent mouillant, un désintégrant, un lubrifiant et un arôme.

7. Utilisation de Lactobacillus plantarum CCFM8661, selon la revendication 3 ou 4, **caractérisé en ce que** ces compositions pharmaceutiques sont de le groupe constitué par
des granules, des capsules, des comprimés, des pilules et des liquides par voie orale.

8. Utilisation de Lactobacillus plantarum CCFM8661, selon la revendication 2, **caractérisé en ce que** ces aliments fermentés comprennent des produits laitiers, des produits de soja et des produits de fruits et de légumes, qui tous sont produit avec un agent de fermentation, comprenant des Lactobacillus plantarum CCFM8661.

9. Utilisation de Lactobacillus plantarum CCFM8661, selon la revendication 8, **caractérisé en ce que** la préparation de ledit agent de fermentation comprend les étapes suivantes:
(A) Préparation d'un milieu de culture:
10% d'une hydrolyse enzymatique du lait écrémé, 0,5% de glucose, 1,5% de tryptone et
0,3% d'extrait de levure sont tous dissous dans l'eau, ce qui représente 87,7% du poids total du milieu de culture, la valeur du pH est ajustée à 6,8;
(B) Préparation du protecteur:
utiliser de l'eau et du matériel de protecteur pour la préparation du protecteur, contenant 100g/l de lait écrémé en poudre, 30mg/l de glycérol, 100 g/l de maltodextrine, 150g/l de tréhalose et 10g/l de L-glutamate de sodium;
(C) Inoculation d'un milieu de culture avec Lactobacillus plantarum CCFM8661, que représente 2-4% du poids du milieu de culture, le milieu de culture a été stérilisé 8-12 min à 110-120°C, puis mise en culture pendant 18 heures à 37°C, puis lavage à pH 7,2 avec PBS 2-4 fois, puis remettre en suspension dans le protecteur jusqu'à la concentration de 1010 UFC/ml; par la suite, pré-culture la suspension pendant 60 minutes à 37°C, et puis lyophilise pour obtenir ledit agent de fermentation.

10. Utilisation de Lactobacillus plantarum CCFM8661, selon la revendication 8, **caractérisé en ce que** ces produits laitiers comprennent du lait de vache, du crème aigre, et du fromage; **en ce que** ces produits de soja comprennent du lait de soja, du sauce homardine, et du sauce de soja fermenté; et **en ce que** ces produits à base de fruits et de légumes comprennent du pruduits de oncombre, de carotte, de la betterave , de céleri et de choux.
